# EUROPÄISCHE PATENTANMELDUNG

(11) **EP 4 480 464 A1**
(43) Veröffentlichungstag der Anmeldung: **25.12.2024**
(21) Anmeldenummer: 24181166.0
(22) Anmeldetag: 10.06.2024
(51) Int. Cl.: A61G 3/00, A61B 6/00

(54) **MOBILE ZAHNARZTPRAXIS**

(30) Priorität: 23.06.2023 EP 23181156
(71) Anmelder: Dentaxis GmbH, 9500 Wil SG (CH)
(72) Erfinder: Birkhoven-Schmidt, Christine, 8004 Zürich (CH); Scheidegger, Pascal, 9555 Tobel (CH); Meyenberger, Patrick, 9500 Wil (CH)
(74) Vertreter: Hepp Wenger Ryffel AG

(57) **Zusammenfassung**

Eine Mobile Zahnarztpraxis (1) umfasst ein Fahrzeugchassis (2) sowie ein Praxismodul (3), wobei das Fahrzeugchassis (2) eine Fahrerkabine (4) umfasst und das Praxismodul (3) auf dem Fahrzeugchassis (2) befestigt ist. Das Praxismodul (3) weist zumindest eine Zugangstür (5) auf, durch die das Praxismodul (3) betretbar ist, wobei das Praxismodul (3) einen Behandlungsraum (6) aufweist. In dem Behandlungsraum (6) ist ein Behandlungsstuhl (7) angeordnet, der durch eine Versorgungseinrichtung (28) mit Energie und/oder Medien versorgbar ist. In der Nähe des Behandlungsstuhls (7) ist ein Röntgengerät (8) angeordnet, so dass auf dem Behandlungsstuhl (7) Röntgenbilder anfertigbar sind.

## Beschreibung

Die Erfindung betrifft eine mobile Zahnarztpraxis.

Insbesondere für Patienten, die nicht gut zu Fuss sind, Mobilitäts- oder anderweitige Einschränkungen haben, ist die zahnmedizinische vor Ort Betreuung notwendig. Hierfür gibt es Behandlungskoffer, die der Zahnarzt mit an den Behandlungsort nehmen kann. Es sind weiterhin auch selbst umgebaute Fahrzeuge bekannt, die in der Art eines fahrenden Koffers notwendige Utensilien transportieren können. Nachteilig an diesen Lösungen ist bisher, dass nicht alle notwendigen Geräte und Instrumente vorhanden sind und dass der Patient vor Ort auf einer Massageliege oder einem anderen Provisorium behandelt werden muss, an dem die Beleuchtung nicht gut ist oder die Position der Patienten für die Behandler ergonomisch nicht gut ist. Gleichzeitig muss ein Fahrzeug durch Jeden, der ein PW-Fahrausweis (Schweiz = Kat. B) besitzt, fahrbar sein und muss daher unter den gesetzlich vorgeschriebenen Gewichtslimiten liegen.

Es ist daher Aufgabe der Erfindung eine mobile Zahnarztpraxis zu schaffen, die die Nachteile des Standes der Technik vermeidet, und die insbesondere eine, einer Zahnarztpraxis ähnliche Umgebung schafft, die von jedem gefahren werden darf und trotzdem sämtliche Utensilien und Instrumente, die für eine Behandlung nach modernem Standard notwendig sind, mitführt sowie eine optimale Behandlung ermöglicht.

Die Aufgabe wird durch eine mobile Zahnarztpraxis gemäss dem unabhängigen Anspruch 1 gelöst.

Insbesondere wird die Aufgabe durch eine mobile Zahnarztpraxis gelöst, die ein Fahrzeugchassis sowie ein Praxismodul umfasst.

Das Fahrzeugchassis umfasst eine Fahrerkabine und das Praxismodul ist auf dem Fahrzeugchassis befestigt. Das Praxismodul weist zumindest eine Zugangstür auf, durch die das Praxismodul betretbar ist. Das Praxismodul weist einen Behandlungsraum auf, wobei in dem Behandlungsraum ein Behandlungsstuhl angeordnet ist, der durch eine Versorgungseinrichtung mit Energie und/oder Medien versorgbar ist. In der Nähe des Behandlungsstuhls ist ein Röntgengerät angeordnet, so dass auf dem Behandlungsstuhl intraorale Röntgenbilder eines Patienten anfertigbar sind.

In einer derartigen mobilen Zahnarztpraxis können die Patienten nahezu so vor Ort behandelt werden wie in einem normalen Praxisraum. Es sind alle Instrumente vorhanden, insbesondere kann auch eine intraorale Röntgenaufnahme angefertigt werden, welche für die Befundung der Zähne nach heutigem Standard notwendig ist.

Das Röntgengerät ist bevorzugt an einem beweglichen Arm an der Wand befestigt und kann so bei der Benutzung in Richtung Patient bewegt werden und zum Transport wieder an der Wand angeordnet werden. Um die Installation eines Röntgengeräts möglich zu machen, muss die Raumhöhe in einem Bereich von 2,2m bis 2,4m liegen. In der Nähe bedeutet, dass das Röntgengerät in einem Radius von maximal 1,5 Metern vom Behandlungsstuhl angeordnet ist, um das ordnungsgemässe Anfertigen eines Röntgenbildes zu ermöglichen. Das Fahrzeugchassis ist bevorzugt ein Aluminium-Niederrahmenchassis, welches die notwendige Festigkeit und Tragfestigkeit mitbringt und gleichzeitig leicht ist, sodass auch bei Installationen von Geräten das Fahrzeug die gesamtzulässigen Masse nicht überschreitet.

Das Praxismodul kann einen Maschinenraum umfassen, wobei der Maschinenraum insbesondere eine Schallabsorptionsschicht umfasst.

Im Maschinenraum sind unter anderem ein Frischwassertank und/oder eine Absaugevorrichtung und/oder eine Kompressorvorrichtung und/oder ein Amalgamabscheider vorgesehen. Zusätzlich kann ein Abwassersammler vorgesehen sein.

Durch die Anordnung der technischen Geräte an einem Maschinenraum sind alle technischen Geräte an einem Ort vorgesehen, was die Wartung erleichtert. Die Leitungen zwischen den Geräten müssen nicht lang sein und der entstehende Schall kann durch die Schalldämmung des Maschinenraums lokal begrenzt werden, so dass die Patienten und Mitarbeitenden durch den entstehenden Schall nicht belästigt werden.

Der Maschinenraum kann von aussen durch die Praxismodulwand durch eine Servicetüre zugänglich sein, wobei insbesondere die Servicetüre in einem geschlossenen Zustand, einen spaltgeöffneten Zustand und einen geöffneten Zustand bringbar ist, wobei die Servicetür im spaltgeöffneten Zustand in ihrer Position fixiert ist.

So kann der Maschinenraum bei Verwendung des Praxismoduls belüftet werden und wird nicht überhitzt, ohne dass eine separate Belüftungsvorrichtung oder Lüftungsschlitze nötig sind, durch die von Aussen Wasser eindringen könnte.

Der Behandlungsraum kann oberhalb des Praxismodulbodens einen Fussboden aufweisen, wobei die Versorgungseinrichtung unterhalb des Fussbodens und oberhalb des Praxismodulbodens verlegt ist.

So sind keinerlei Stolperfallen vorhanden und der Boden ist eben und sicher. Durch die Anordnung der Geräte im Maschinenraum ist weiterhin die notwendige Leitungslänge sehr begrenzt und es wird Gewicht eingespart.

Der Behandlungsstuhl und der Maschinenraum sind an dem Praxismodulboden befestigt.

Somit entstehen keine zusätzlichen, und insbesondere verstärkten, Schwingungen, die unerwünschte Abrieb-, Verschleiss- oder Lockerungserscheinungen der Verbindungen verursachen könnten.

Die Versorgungseinrichtung umfasst eine, insbesondere isolierte, Absaugverbindung. Zusätzlich kann die Versorgungseinrichtung eine Wasserversorgungsleitung, eine Druckluftversorgungsleitung sowie bevorzugt eine elektrische Versorgungsleitung umfassen. Somit können alle vom Behandlungsstuhl benötigten Medien hin- und auch wieder zurücktransportiert werden. Eine Isolierung stellt sicher, dass auch bei kalten Aussentemperaturen die Absaugeverbindung sicher funktioniert.

Das Praxismodul kann eine Decke aufweisen, wobei unterhalb der Praxismoduldecke eine Akustikdecke angeordnet ist, wobei die Akustikdecke insbesondere eine Öffnung für eine Raumbeleuchtung aufweist.

Durch die Akustikdecke wird die Schallverbreitung innerhalb des Praxismoduls erschwert und somit die Behandlungsqualität für den Patienten verbessert. Die Akustikdecke basiert bevorzugt auf einer Rippenkonstruktion und ist zweischichtig aufgebaut. Die Untere, abgehängte Schicht hat eine Ovale Öffnung und die obere Schicht innerhalb der ovalen Öffnung ist direkt an die Decke montiert. Weiterhin ist die Akustikdecke mit einer Schattenfuge von der Wand bzw. den Wänden beabstandet, um eine relative Bewegung der Akustikdecke zum Praxismodul während der Fahrt zu ermöglichen und die Stabilität der Decke zu gewährleisten.

Das Praxismodul kann eine Klimaanlage und/oder eine Standheizung umfassen. Somit kann immer ein optimales Klima innerhalb des Praxismoduls gewährleistet werden. Hierbei kann die Standheizung ausserhalb des Praxismoduls, bevorzugt aussen am Chassis, insbesondere unterhalb der Fahrerkabine, angeordnet sein. Zusätzlich kann ein separater Maschinenraum-Klimatisierungskanal ausgebildet sein, der von Klimaanlage und/oder Standheizung zum Maschinenraum reicht und auch im Winter so eine konstante Temperatur auch des Maschinenraums gewährleistet.

Somit arbeiten die Maschinen effizient und sicher.

Das Praxismodul kann einen in Fahrtrichtung vorderen und einen in Fahrtrichtung hinteren Bereich aufweisen, wobei der Behandlungsstuhl im hinteren Bereich und der Maschinenraum im vorderen Bereich angeordnet ist.

Somit sind die schweren Teile des Praxismoduls entlang der Längsachse des Fahrzeuges beabstandet angeordnet und das Gewicht ist gleichmässig verteilt.

Das Praxismodul kann einen in Fahrtrichtung ersten Seitenbereich und einen in Fahrtrichtung zweiten Seitenbereich aufweisen, wobei der Behandlungsstuhl, und insbesondere das Röntgengerät, im ersten Seitenbereich und der Maschinenraum im zweiten Seitenbereich angeordnet sind.

Somit sind auch in Querrichtung die schweren Geräte beabstandet angeordnet und auch in Querrichtung ist das Fahrzeug aus Gewichtssicht balanciert und gleichmässig belastet.

Das Chassis kann ein Hubstützensystem umfassen, durch die die mobile Zahnarztpraxis im Wesentlichen ungefedert fixierbar ist.

Auch kann durch das Hubstützensystem die mobile Zahnarztpraxis nivelliert werden, so dass ein optimales Arbeiten möglich ist.

Somit werden Schwingungen, die während der Behandlung entstehen, nicht gefedert und das Chassis steht sicher und fest ohne Vibration an einem Ort. Ungefedert bedeutet, dass keine zusätzliche Federung vorhanden ist. Die vorhandene elastische Verformbarkeit der Bauteile ist keine zusätzliche Federung.

Möbel für den Transport und die Lagerung von Material und Instrumenten sind in dem Praxismodul befestigt, insbesondere auf dem Praxismodulboden befestigt, wobei die Möbelfronten zumindest teilweise eine Kombination von Aluminium-Lochblech und PET-Panel umfassen.

Somit können alle Materialien und Instrumente, die für die Behandlung notwendig sind, sicher in dem Praxismodul angeordnet werden, sind schnell auffindbar und während des Transports sicher untergebracht. Gleichzeitig erscheint die mobile Zahnarztpraxis den Patienten aufgeräumt und durch die spezielle Materialwahl der Möbelfronten ist das Fahrzeug noch immer leicht genug, um nicht die 3,5t Grenze zu überschreiten.

Die Möbel können im vorderen Bereich, insbesondere an der Stirnseite, des Praxismoduls angeordnet sein und insbesondere ist auch der Maschinenraum in einem Möbel angeordnet.

Somit ist der hintere Bereich des Praxismoduls möbelfrei ausgebildet, so dass dort Platz für Patienten, Behandlungsstuhl und eine weitere Zugangstür ist.

Das Praxismodul kann eine zweite Zugangstür umfassen, die an der hinteren Stirnseite angeordnet ist, und insbesondere einen Rollstuhllift umfasst.

Somit können Patienten leicht das Praxismodul betreten und es ist auch für Patienten mit reduzierter Mobilität möglich, den Behandlungsraum zu betreten.

Das Praxismodul kann eine innere Raumhöhe von 2.1m bis 2.3m, insbesondere 2.18m, aufweisen. Die innere Raumhöhe ist die Raumhöhe bis zur Akustikdecke.

Durch eine derartige Deckenhöhe können alle Geräte gut untergebracht werden, und die Menschen, die den Praxisraum betreten, können dort ohne Probleme stehen.

Das für die Möbel notwendige Möbelgerüst kann aus Leichtbauplatten konstruiert sein. Insbesondere sind feuchtigkeitsresistent verleimte Albasiaholzplatten wie beispielsweise Queenply vorgesehen.

Das Praxismodul kann eine Länge von im Wesentlichen 4m aufweisen. Die mobile Praxis kann ein maximales Gesamtgewicht von 3,5t aufweisen.

Somit kann genug Platz geschaffen werden, um alle notwendigen Praxisgeräte anzuordnen. Weiterhin ist das Fahrzeug mit einem normalen Führerschein fahrbar.

Lüftungsschlitze der Klimaanlage und/oder der Standheizung können im Bereich der Stirnwand in den Möbelfronten im Bereich der oberen Decke und/oder im Bereich des Fussbodens angeordnet sein. Somit wird eine optimale Klimatisierung gewährleistet.

Der Behandlungsstuhl umfasst insbesondere keine separate Armleuchte. Dies führt zu einer weiteren Gewichtsersparnis.

Im Folgenden wird die Erfindung an Hand von Ausführungsbeispielen in Figuren näher erläutert. Hierbei zeigt:
- Figur 1:: Einen Schnitt durch eine mobile Zahnarztpraxis,
- Figur 2:: eine perspektivische Seitenansicht einer mobilen Zahnarztpraxis,
- Figur 3:: eine weitere perspektivische Seitenansicht einer mobilen Zahnarztpraxis,
- Figur 4:: zeigt eine Innenansicht einer mobilen Zahnarztpraxis,
- Figur 5:: zeigt eine weitere Innenansicht der mobilen Zahnarztpraxis,
- Figur 6:: zeigt ein Detail der Decke des Praxismoduls,
- Figur 7:: zeigt den vorderen Bereich des Praxismoduls,
- Figur 8:: zeigt einen weiteren vorderen Bereich des Praxismoduls.

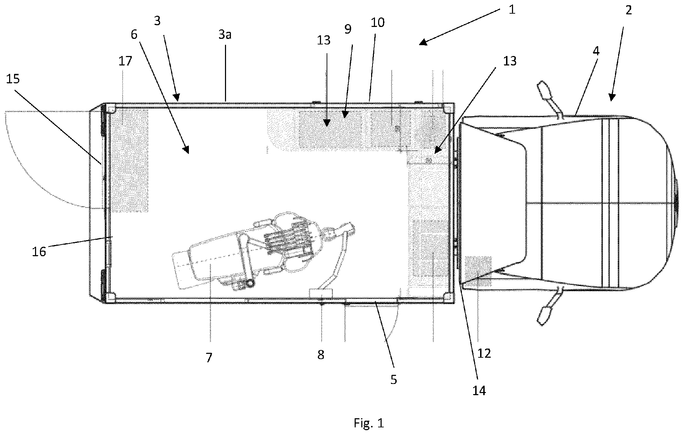

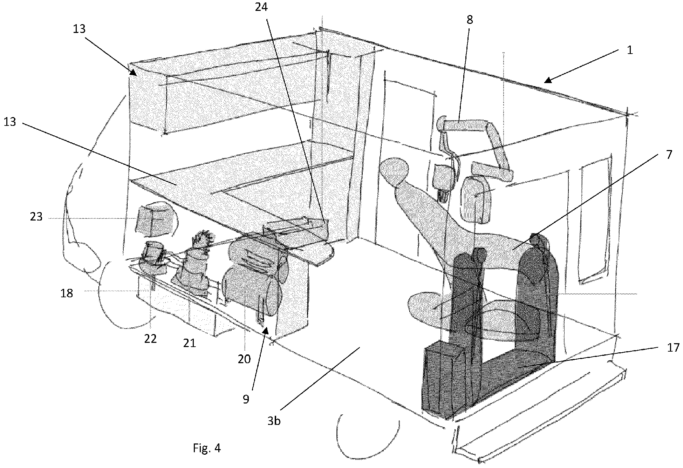

Figur 1 zeigt einen Schnitt durch eine mobile Zahnarztpraxis 1, die ein Fahrzeugchassis 2 sowie ein Praxismodul 3 umfasst. Das Fahrzeugchassis ist auf Rädern angeordnet. Das Praxismodul 3 ist auf dem Fahrzeugchassis 2 befestigt, wobei das Fahrzeugchassis 2 zusätzlich eine Fahrerkabine 4 umfasst. Das Praxismodul 3 umfasst einen Behandlungsstuhl 7, der im Behandlungsraum 6 angeordnet ist. Der Behandlungsraum 6 ist durch die Zugangstür 5 betretbar. In der Nähe des Behandlungsstuhls 7 ist ein Röntgengerät 8 an der Seitenwand des Praxismoduls 3 befestigt und über einen Arm in die richtige Position für den Patienten bringbar. Der Behandlungsstuhl 7 ist im hinteren Bereich des Praxismoduls 3 befestigt, wobei Möbel 13 im vorderen Bereich des Praxismoduls 3 angeordnet sind. Der Behandlungsstuhl 7 weist keine Armleuchte auf. In den Möbeln 13 an der Stirnseite 14 sind Schubladen für Instrumente und Geräte sowie Materialien untergebracht. Die Schubladen und Türen der Möbel 13 umfassen jeweils eine zusätzliche Öffnungssicherung zur Verhinderung von Öffnen während der Fahrt. Weiterhin ist dort eine Arbeitsfläche angeordnet. Im ersten seitlichen Bereich sind der Behandlungsstuhl 7 und das Röntgengerät 8 angeordnet, wobei im zweiten Seitenbereich der Maschinenraum 9in einem Möbel 13 angeordnet ist. Im Maschinenraum 9 sind die unterschiedlichen notwendigen Maschinen wie Kompressor, Absaugevorrichtung, Frischwassertank, Amalgamabscheider sowie Abwassersammler (s. Fig. 4) angeordnet. Der Maschinenraum 9 ist weiterhin schallisoliert ausgebildet, so dass etwaige Schallbelastungen aus dem Maschinenraum in Richtung Patient gedämpft sind. An der hinteren Stirnseite 16 des Praxismoduls 3 ist weiterhin eine zweite Zugangstür 15 angeordnet, die breiter ausgebildet ist als die erste Zugangstür 5, so dass auch Rollstuhlfahrer den Praxisraum betreten können. Hierfür ist an der hinteren Stirnseite ein Rollstuhllift (17) oder eine andere Zugangsmöglichkeit wie beispielsweise eine Rampe für Rollstuhlfahrer vorgesehen. Der Maschinenraum 9 ist durch die Seitenwand 3a des Praxismoduls 3 durch eine Servicetür 10 zugänglich. Die Servicetür 10 kann im Stillstand während der Behandlung in einer spaltgeöffneten Position fixiert werden, so dass der Maschinenraum 9 belüftet ist. Der Behandlungsraum 6 ist durch eine Klimatisierung klimatisiert, wobei die Standheizung 12 ausserhalb des Praxismoduls 3 angeordnet ist, bevorzugt unter der Fahrerkabine. Das maximale Gewicht der mobilen Zahnarztpraxis ist 3.5t.

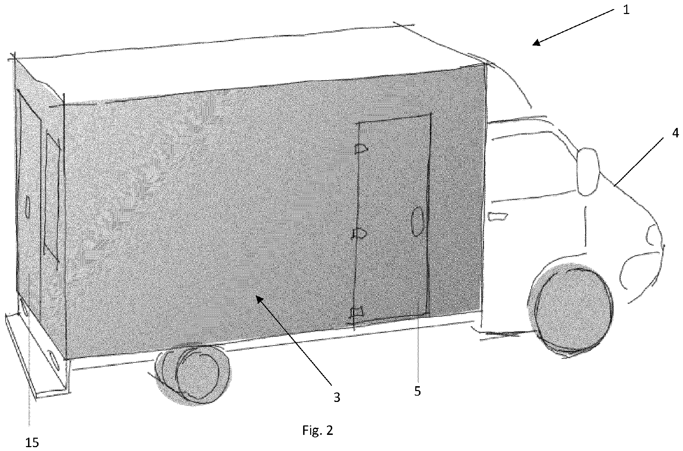

Figur 2 zeigt die mobile Zahnarztpraxis 1 in einer Aussenansicht. Die mobile Zahnarztpraxis 1 entspricht der mobilen Zahnarztpraxis 1 aus Figur 1. An der Aussenansicht ist die Fahrerkabine 4 ersichtlich, die Teil des Fahrzeugschassis 2 ist und welches auf zwei Achsen mit Rädern gelagert ist. Das Praxismodul 3 weist eine Zugangstür 5 auf, durch die das Praxismodul 3 betretbar ist. Eine zweite Zugangstür 15 ist an der hinteren Stirnseite angeordnet, und ist breiter ausgebildet als die erste Zugangstür 5.

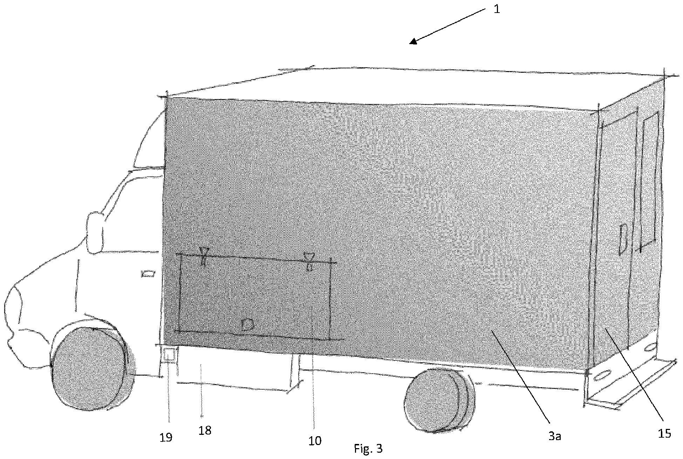

Figur 3 zeigt eine weitere Aussenansicht der mobilen Zahnarztpraxis 1 von der anderen Seite im Vergleich zu Figur 2. Die Ausführungsform entspricht jedoch weiterhin den Figuren 1 und 2. Das Praxismodul 3 hat in seiner Praxismodulwand 3a die Serviceklappe 10, die zum Maschinenraum führt. Unterhalb des Maschinenraums 9 und der Servicetür 10 ist ein Abwassertank 18 angeordnet. Der Abwassertank 18 sammelt die bei der Behandlung auftretenden abgesaugten Abwässer. Weiterhin ist ein Stromanschluss 19 ausgebildet, durch den das Fahrzeug von Extern mit Strom versorgbar ist. Bevorzugt ist der Stromanschluss ein 16 A Stromanschluss, so dass die Stromstärke für die Versorgung aller Geräte sicher ausreicht.

Figur 4 zeigt den Innenraum der mobilen Zahnarztpraxis 1. In dieser perspektivischen Ansicht des Innenraums, die der Ausführungsform in Figur 1 entspricht, sind die Möbel 13 sowie die Maschinen im Maschinenraum 9 klar erkennbar. Im Maschinenraum 9 sind ein Kompressor 20, ein Absauger 21, ein Amalgamabscheider 22 sowie eine Frischwasserversorgung 23 angeordnet. Unterhalb des Maschinenraums 9 befindet sich weiterhin der Abwassertank 18. Die Klimaanlage 24 ist an der vorderen Stirnseite 14 im unteren Bereich des Möbels 13 angeordnet. Der Behandlungsstuhl 7, das Röntgengerät 8 sowie der Rollstuhllift 17 sind im hinteren Bereich des Praxismoduls 3 angeordnet. Das Praxismodul 3 weist ein Praxismodulboden 3b auf, an dem der Behandlungsstuhl 7 und die Maschinen im Maschinenraum 9 befestigt sind. Die Höhe des Praxismoduls 3 ist so ausgerichtet, dass ein Röntgengerät 8 zulässig ist.

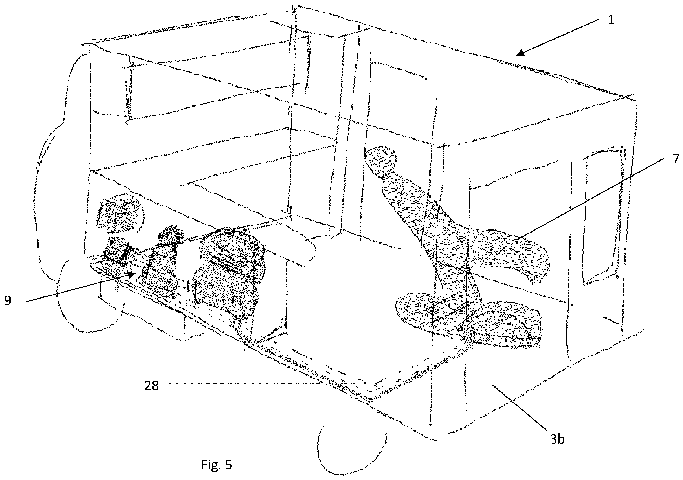

Figur 5 zeigt eine weitere perspektivische Innenansicht der mobilen Zahnarztpraxis 1 gemäss Figur 1. Es ist hier die Versorgungseinrichtung 28 gezeigt, die vom Maschinenraum 9 zum Behandlungsstuhl 7 verläuft. Die Versorgungseinrichtung 28 umfasst eine isolierte Absaugerverbindung sowie Druckluft, Strom und Frischwasserleitung. Die Versorgungseinrichtung 28 ist oberhalb des Praxismodulbodens 3b angeordnet und durch einen Fussboden aus Linoleum (nicht sichtbar) abgedeckt. Der Behandlungsstuhl 7 ist ebenfalls auf dem Praxismodulboden 3b befestigt.

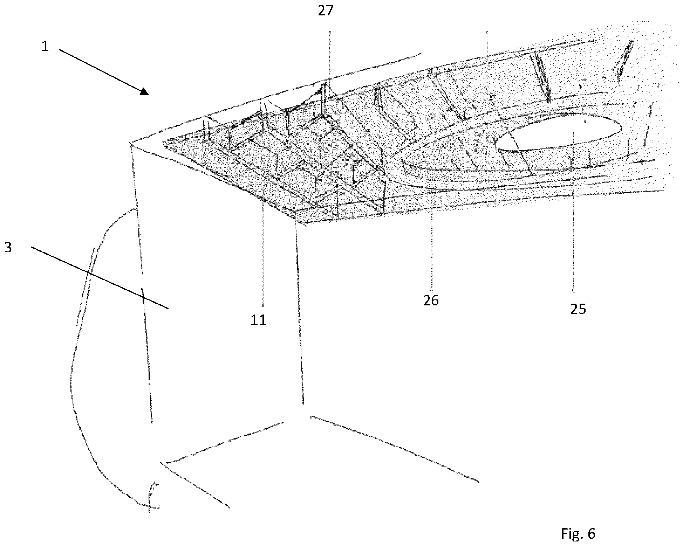

Figur 6 zeigt die Deckenkonstruktion des Praxismoduls 3 der mobilen Zahnarztpraxis 1 aus Figur 1. Die Akustikdecke 11 umfasst einen Akustikschaumstoff aus recyceltem PET und absorbiert somit Schall optimal. Die Akustikdecke 11 weist eine Rippenkonstruktion 27 auf und ist somit sehr leicht. Die abgehängte Akustikdecke 11 ist in zwei Hälften konzipiert und verfügt über eine Schattenfuge von etwa 1cm bis 2cm Breite entlang der Praxismodulwand. Die Akustikdecke 11 weist eine Öffnung auf, in der eine Leuchte 25 und ein, bevorzugt in Farbe und Lichtstärke variierbares, LED-Band 26 angeordnet sind. Somit ist eine optimale Beleuchtung oberhalb des Behandlungsstuhls 7 ermöglicht.

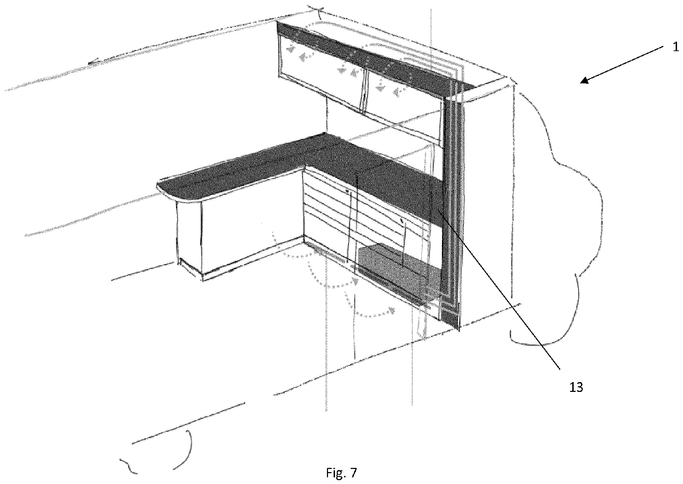

Figur 7 zeigt den Verlauf der Belüftungs- und Klimatisierungskanäle durch die Möbel 13 in der mobilen Zahnarztpraxis 1 gemäss Figur 1. Oberhalb der Möbelfront 13 sind Aussparungen angeordnet, die im Wesentlichen für den Patienten nicht sichtbar sind. Durch diese Aussparungen in den Fronten der Möbel 13 kann klimatisierte Luft von der Klimaanlage in den Behandlungsraum 6 eingeführt werden. Weitere Lüftungsschlitze durch Aussparungen in den Möbelfronten sind oberhalb des Fussbodens in der Möbelfront im unteren Bereich des Möbels 13 ausgebildet.

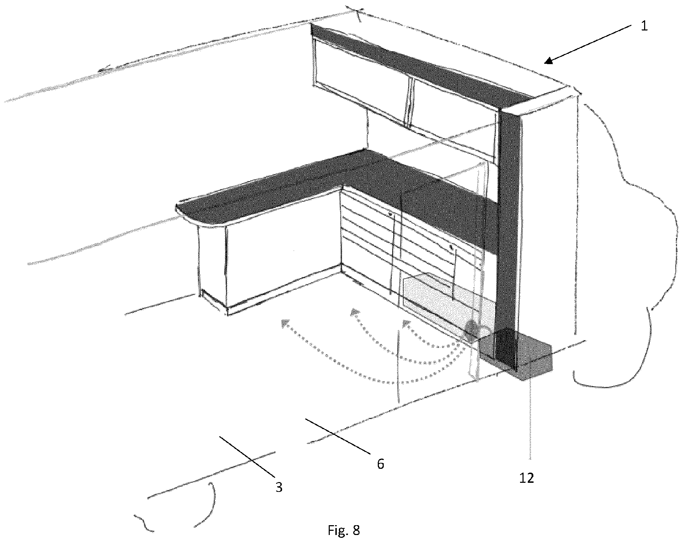

Figur 8 zeigt die mobile Zahnarztpraxis 1 aus Figur 1 mit Darstellung der Standheizung 12. Die Standheizung 12 ist unterhalb des Fahrerhauses angeordnet und weist eine Leitung von aussen in den Behandlungsraum 6 des Praxismoduls 3 auf.

## Patentansprüche

1. Mobile Zahnarztpraxis (1) umfassend ein Fahrzeugchassis (2) sowie ein Praxismodul (3), wobei das Fahrzeugchassis (2) eine Fahrerkabine (4) umfasst und das Praxismodul (3) auf dem Fahrzeugchassis (2) befestigt ist, wobei das Praxismodul (3) zumindest eine Zugangstür (5) aufweist, durch die das Praxismodul (3) betretbar ist, wobei das Praxismodul (3) einen Behandlungsraum (6) aufweist, wobei in dem Behandlungsraum (6) ein Behandlungsstuhl (7) angeordnet ist, der durch eine Versorgungseinrichtung (28) mit Energie und/oder Medien versorgbar ist, wobei in der Nähe des Behandlungsstuhls (7) ein Röntgengerät (8) angeordnet ist, so dass auf dem Behandlungsstuhl (7) intraorale Röntgenbilder anfertigbar sind.

2. Mobile Zahnarztpraxis (1) nach Anspruch 1, **dadurch gekennzeichnet, dass** das Praxismodul (3) einen Maschinenraum (9) umfasst, wobei der Maschinenraum (9) insbesondere eine Schallabsorptionsschicht umfasst.

3. Mobile Zahnarztpraxis (1) nach Anspruch 2, **dadurch gekennzeichnet, dass** der Maschinenraum (9) von aussen durch die Praxismodulwand (3a) durch eine Servicetür (10) zugänglich ist, wobei insbesondere die Servicetür (10) in einen geschlossenen Zustand, einen spaltgeöffneten Zustand und einen geöffneten Zustand bringbar ist, wobei die Servicetür (10) im spaltgeöffneten Zustand in ihrer Position fixierbar ist.

4. Mobile Zahnarztpraxis (1) nach einem der vorhergehenden Ansprüche, **dadurch gekennzeichnet, dass** der Behandlungsraum (6) oberhalb des Praxismodulbodens (3b) einen Fussboden aufweist, wobei die Versorgungseinrichtung (28) unterhalb des Fussbodens und oberhalb des Praxismodulbodens (3b) verlegt ist.

5. Mobile Zahnarztpraxis (1) nach Anspruch 4, **dadurch gekennzeichnet, dass** der Behandlungsstuhl (7) und der Maschinenraum (9) an dem Praxismodulboden (3b) befestigt sind.

6. Mobile Zahnarztpraxis (1) nach einem der vorhergehenden Ansprüche, **dadurch gekennzeichnet, dass** die Versorgungseinrichtung (28) eine, insbesondere isolierte, Absaugerverbindung umfasst.

7. Mobile Zahnarztpraxis (1) nach einem der vorhergehenden Ansprüche, durch gekennzeichnet, dass das Praxismodul (3) eine Decke (8a) aufweist, wobei unterhalb der Praxismoduldecke (8a) eine Akustikdecke (11) angeordnet ist, wobei die Akustikdecke (11) insbesondere eine Öffnung für eine Raumbeleuchtung aufweist.

8. Mobile Zahnarztpraxis (1) nach einem der vorhergehenden Ansprüche, **dadurch gekennzeichnet, dass** das Praxismodul (3) eine Klimaanlage und/oder eine Standheizung (12) umfasst.

9. Mobile Zahnarztpraxis (1) nach einem der vorhergehenden Ansprüche, **dadurch gekennzeichnet, dass** das Praxismodul (3) einen in Fahrtrichtung vorderen und einen in Fahrtrichtung hinteren Bereich aufweist, wobei der Behandlungsstuhl (7) im hinteren Bereich und der Maschinenraum (9) im vorderen Bereich angeordnet ist.

10. Mobile Zahnarztpraxis (1) nach einem der vorhergehenden Ansprüche, **dadurch gekennzeichnet, dass** das Praxismodul (3) einen in Fahrtrichtung ersten Seitenbereich und einen in Fahrtrichtung zweiten Seitenbereich aufweist, wobei der Behandlungsstuhl (7), und insbesondere das Röntgengerät (8), im ersten Seitenbereich und der Maschinenraum (9) im zweiten Seitenbereich angeordnet ist.

11. Mobile Zahnarztpraxis (1) nach einem der vorhergehenden Ansprüche, **dadurch gekennzeichnet, dass** das Chassis (2) ein Hubstützensystem umfasst, durch welches die mobile Zahnarztpraxis im Wesentlichen ungefedert fixierbar ist.

12. Mobile Zahnarztpraxis (1) nach einem der vorhergehenden Ansprüche, **dadurch gekennzeichnet, dass** Möbel (13) für den Transport und die Lagerung von Material und Instrumenten in dem Praxismodul (3) befestigt sind, insbesondere auf dem Praxismodulboden (3a) befestigt sind, wobei die Möbelfronten (13a) zumindest teilweise eine Kombination von Aluminium-Lochblech und PET-Paneel umfassen.

13. Mobile Zahnarztpraxis (1) nach Anspruch 12, **dadurch gekennzeichnet, dass** die Möbel (13) im vorderen Bereich, insbesondere der vorderen Stirnseite (14), des Praxismoduls (3) angeordnet sind und insbesondere auch der Maschinenraum (9) in einem Möbel (13) angeordnet ist.

14. Mobile Zahnarztpraxis (1) nach einem der vorhergehenden Ansprüche, **dadurch gekennzeichnet, dass** das Praxismodul (3) eine zweite Zugangstür (15) umfasst, die an der hinteren Stirnseite (16) angeordnet ist und insbesondere einen Rollstuhllift (17) umfasst.

15. Mobile Zahnarztpraxis (1) nach einem der vorhergehenden Ansprüche, **dadurch gekennzeichnet, dass** das Praxismodul (3) eine innere Raumhöhe von 2.1m bis 2.3m, insbesondere 2.18m aufweist.
